(19) Europäisches Patentamt
European Patent Office
Office européen des brevets

(11) Veröffentlichungsnummer: **0 264 038**
**A1**

# (12) EUROPÄISCHE PATENTANMELDUNG

(21) Anmeldenummer: **87114455.6**

(51) Int. Cl.⁴: **A61K 31/665**, C07F 9/53

(22) Anmeldetag: **03.10.87**

(30) Priorität: **11.10.86 DE 3634745**

(43) Veröffentlichungstag der Anmeldung: **20.04.88 Patentblatt 88/16**

(84) Benannte Vertragsstaaten: **CH DE FR GB IT LI**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Möller, Hinrich, Dr.**
**Schumannstrasse 11**
**D-4019 Monheim(DE)**

## (54) Glycidylgruppen enthaltende Cytostatika.

(57) Verwendung von Glycidylphosphinoxiden der allgemeinen Formel

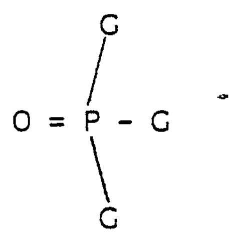

wobei

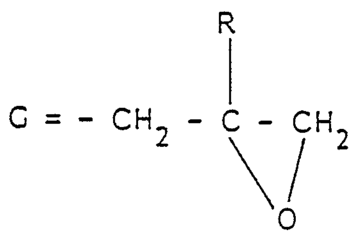

und anstelle eines G der obigen Bedeutung auch ein G' der Bedeutung

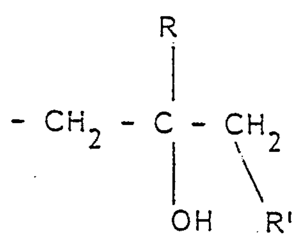

stehen kann,
wobei R H oder Methyl und R' H, Methoxy, Ethoxy oder Halogen bedeuten kann, als Arzneimittel mit cytostatischer Wirksamkeit, wobei insbesondere Triglycidylphosphinoxid bevorzugt ist.

EP 0 264 038 A1

Xerox Copy Centre

## "Glycidylgruppen enthaltende Cytostatika"

Es ist bekannt, daß eine Reihe alkylierender Substanzen, zu denen unter anderem die Stickstofflostderivate gehören, eine cytostatische bzw. cytotoxische Wirkung entfalten. Darüber hinaus ist bekannt, wenigstens 2 Epoxidgruppen im Molkül enthaltende Verbindungen als Cancerostatika zu verwenden. Als Beispiele für derartige Substanzen seien 4,4'-Bis(2,3-epoxypropyl)dipiperidinyl-1,1' und 1,2-15, 16-Diepoxy-4,7,10,13-tetraoxahexadecan genannt. Allerdings haben die letzteren Verbindungen keine wesentliche Verbesserung in der cytostatischen Behandlung gebracht und werden daher kaum verwendet.

Bekannt sind ferner die auf N-haltigen aromatischen Heterocyclen basierenden Cytostatika wie Triglycidylisocyanurate und Triglycidylurazole sowie deren Derivate.

Aufgabe der vorliegenden Erfindung war es, die Reihe der 3 Glycidylgruppen pro Molekül enthaltenden cytostatisch wirksamen Heterocyclen um zusätzliche Vertreter zu erweitern, um so weitere Varianten bei der cytostatischen Behandlung zu ermöglichen.

Gegenstände der vorliegenden Erfindung ist die Verwendung von Glycidylphosphinoxiden der allgemeinen Formel

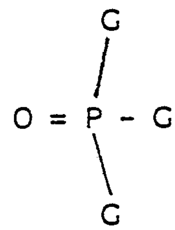

wobei

G =
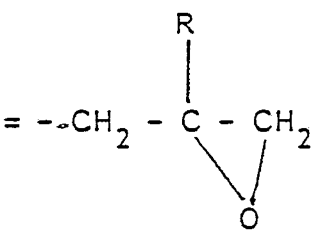

und

anstelle eines G der obigen Bedeutung auch ein $G^1$ der Bedeutung

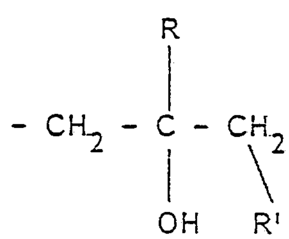

stehen kann,

wobei R H oder Methyl und $R^1$ H, Methoxy, Ethoxy oder Halogen bedeuten kann, als Arzneimittel mit cytostatischer Wirksamkeit.

Bei den vorstehenden Verbindungen handelt es sich um solche die nach bekannten Methoden prinzipiell herstellbar sind (vergl. TAPPI 44 (10) 185 A-189 A (1961)). Eine Methode geht beispielsweise von Trialkylphosphinoxid aus, das mit Percarbonsäure wie m-Chlor-perbenzoesäure in das Triglycidylphosphinoxid überführt werden kann. In anderen Fällen bietet sich die Abspaltung von Halogenwasserstoff aus Halogenhydrin beispielsweise mittels Alkali unter Ausbildung des Glycidylrestes an. Von Triglycidylphosphinoxid ausgehend kann man gezielt einen der Substituenten G durch hydrolytische Spaltung des Epoxidringes oder durch Umsetzung mit Methanol oder Ethanol in den Substituenten G[1] überführen. Soll R in der vorstehenden Formel die Bedeutung $CH_3$ haben, ist bereits bei der Synthese des Phosphinoxides das Ausgangsmaterial entsprechend zu wählen, d. h. beispieslweise anstelle des Alkylmagnesiumbromids eine Grignardverbindung, bei der am C-2 eine Methylgruppe vorhanden ist.

Der Einsatz von Triglycidylphosphinoxid selbst ist aus praktischen Erwägungen bevorzugt. Zur Verwendung als Cancerostatika sollten die Wirksubstanzen mittels geeigneter, pharmakologisch akzeptabler Vehikel appliziert werden. Hierzu eignen sich die üblichen Hilfs-bzw. Trägerstoffe für pharmakoligische Zubereitungen. Im vorliegenden Fall bietet sich die Verwendung wäßriger Systeme, gegebenenfalls zusammen mit verträglichen Glykolethern, wie Glykolmonoethylether oder Butylenglykolmethylether oder Propylenglykolmethylether an, insbesondere wenn der Wirkstoff parenteral appliziert werden soll. Bei oraler Verabreichung sind die pharmazeutisch üblichen Hilfs-bzw. Trägerstoffe anwendbar, sofern sie eine entsprechende Verträglichkeit mit den Glycidylverbindungen aufweisen.

Die erfindungsgemäß beschriebenen Glycidylverbindungen sollten in den Arzneimittelgemischen üblicherweise in Konzentrationen bis zu 20 Gew.-% - bezogen auf die Arzneimittelmischung - vorliegen. Bevorzugt wird der Bereich zwischen 0,05 und 10 Gew.-% sein.

## Herstellungsbeispiele des Triglycidylphosphinoxid

Die Lösung von 2,4 g (0,015 Mol) Triallylphosphinoxid und 14,0 g (0,073 Mol) m-Chlorperbenzoesäure in 100 ml Methylenchlorid wurden 9 Tage bei Raumtemperatur gerührt. Nach Abfiltrieren der ausgefallenen m-Chlorbenzoesäure wurde die Lösung an Kieselgel (Kieselgel 60 Körnung 0,063 mm für Säulenchromatographie, Merck) chromatographiert (Elutionsmittel: Mehtylenchlorid/Ethylacetat/Methanol (6:4:1)). Die Fraktionen, die das Reaktionsprodukt ($R_f$-Wert ist kleiner als der des Ausgangsproduktes) enthielten, wurden ein 2. Mal mit dem gleichen System chromatographiert. Die Endprodukte enthaltenden Fraktionen wurden auf Peroxide geprüft und bei positivem Befund an einer Säule mit stark barischem Aluminiumoxid (Elutionsmittel wie oben) chromatographiert. Die jetzt peroxidfreien Fraktionen mit chromatographisch reinem Endprodukt wurden vereinigt, zur Trockne gedampft und im Hochvakuum getrocknet.

Es wurden 0,859 Triglycidylphosphinoxid als farblose Flüssigkeit erhalten.

Der Phosphorgehalt wurde mit 13,3% bestimmt (ber. 14,2 %).

Epoxid-O: berechnet 22 %, gefunden 20 %.

Das Massen-, IR-und NMR-Spektrum stützen die Struktur.

## Bestimmung der cytostatischen Wirksamkeit

Die nachfolgenden Versuche wurden durchgeführt nach Testvorschriften des National Cancer Institute Bethesda, Maryland 200014, veröffentlicht in "Cancer Chemotherapy Reports" Part. 3, Sept. 1972, Vol. 3, Nr. 2.

Die Substanz wurde als wäßrige 1 %ige Injektionslösung unmittelbar vor der Applikation frisch hergestellt.

Bei Mäusen wurden gemäß Protokoll 1200 (S. 91 c) die Tumorart. P 388 (Leukämie) i.p. mit $10^6$ Zellen/Maus gesetzt. Die unbehandelten Tiere hatten eine mittlere Überlebensdauer von 10 Tagen.

Mit den behandelten Testtieren, die einer 9-tägigen i.p. Behandlung mit der in Tabelle 1 angegebenen Dosis unterworfen wurden, wurde eine signifikante Verlängerung der Lebensdauer gegenüber der mittleren Überlebensdauer der nicht mit dem Wirkstoff behandelten Tiere erzielt. Die Verlängerungsrate T/C in Abhängigkeit von der Dosierung des Wirkstoffes ist nachstehend tabellarisch zusammengestellt.

Verlängerungsrate T/C in %

| Dosis | 15 | 7,5 | 3,75 | (mg/kg) |
|---|---|---|---|---|
| T/C | 243 | 176 | 163 | (%) |

**Ansprüche**

1. Verwendung von Glycidylphosphinoxiden der allgemeinen Formel

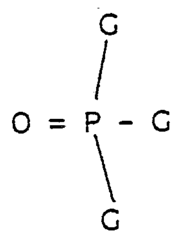

wobei

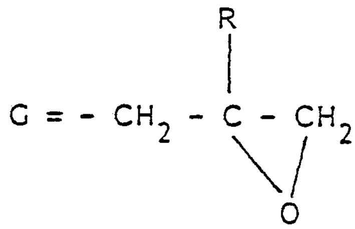

und anstelle eines G der obigen Bedeutung auch ein $G^1$ der Bedeutung

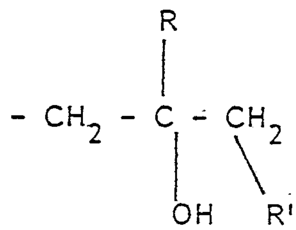

stehen kann,
wobei R H oder Methyl und $R^1$ H, Methoxy, Ethoxy oder Halogen bedeuten kann, als Arzneimittel mit cytostatischer Wirksamkeit.

2. Verwendung von Triglycidylphosphinoxid gemäß Anspruch 1 in pharmakologisch akzeptablen Vehikeln.

Europäisches Patentamt

# EUROPÄISCHER RECHERCHENBERICHT

Nummer der Anmeldung

EP 87 11 4455

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EUROPEAN JOURNAL OF MEDICINAL CHEMISTRY - CHIMIE THERAPEUTIQUE, Band XIX, Nr. 4, 1984, Seiten 331-340; G. SOSNOVSKY et al.: "In the search for new anticancer drugs. VIII Oxaziridines and epoxides" * Seite 335, Tabelle I; Seite 339, "preparations" 41,42 * ----- | 1,2 | A 61 K 31/665<br>C 07 F 9/53 |
| | | | **RECHERCHIERTE SACHGEBIETE (Int. Cl.4)** |
| | | | C 07 F 9/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 20-11-1987 | BESLIER L.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)